# FASCICULE DE BREVET EUROPEEN

(11) **EP 2 796 191 B1**
(45) Date de publication et mention de la délivrance du brevet: **25.12.2024**
(21) Numéro de dépôt: 14170429.6
(22) Date de dépôt: 24.07.2009
(51) Int. Cl.: C12M 1/00, C12M 1/34, G01N 27/22, G01N 33/487

(54) **Bioréacteur avec une paroi percée portant un tube récepteur avec un dispositif capteur à usage unique**
Bioreaktor mit einer durchgebrochenen Wand, durch die ein Aufnahmerohr mit einer Sensorvorrichtung zum einmaligen Gebrauch verläuft
Bioreactor with a bored wall supporting a receiving tube with a disposable sensor device

(30) Priorité: 25.07.2008 FR 0855138
(43) Date de publication de la demande: 29.10.2014
(62) Demande divisionnaire de: 09740390.1
(73) Titulaire: Hamilton Bonaduz AG, 7402 Bonaduz (CH)
(72) Inventeur: Ossart, Frédéric, 30980 Langlade (FR)
(74) Mandataire: Ruttensperger Lachnit Trossin Gomoll

(56) Documents cités:
- WO-A1-2005/068059
- WO-A2-02/102961
- FR-A1- 2 867 278
- JOHN P CARVELL ET AL: "On-line Measurements and Control of Viable Cell Density in Cell Culture Manufacturing Processes using Radio-frequency Impedance", CYTOTECHNOLOGY, KLUWER ACADEMIC PUBLISHERS, DO, vol. 50, no. 1-3, 23 June 2006 (2006-06-23), pages 35 - 48, XP019393895, ISSN: 1573-0778, DOI: 10.1007/S10616-005-3974-X
- DAVEY C L ET AL: "REAL-TIME MONITORING OF THE ACCRETION OF RHIZOPUS OLIGOSPORUS BIOMASS DURING THE SOLID-SUBSTRATE TEMPE FERMENTATION", vol. 7, no. 2, 1 March 1991 (1991-03-01), pages 248 - 259, XP003033463, ISSN: 0959-3993, Retrieved from the Internet <URL:http://rd.springer.com/article/10.1007%2FBF00328998#> DOI: 10.1007/BF00328998
- K. ASAMI ET AL: "Dielectric spectroscopy of biological cells", BIOELECTROCHEMISTRY AND BIOENERGETICS, vol. 40, no. 2, 1 August 1996 (1996-08-01), pages 141 - 145, XP055142392, ISSN: 0302-4598, DOI: 10.1016/0302-4598(96)05067-2

## Description

La présente demande de brevet décrit des dispositifs capteurs de biomasse à usage unique.

La présente invention concerne un bioréacteur ou conteneur selon le terme générique de la revendication 1. L'élaboration de nouvelles molécules par voie biotechnologique est toujours considérée comme l'axe principal de développement pharmaceutique. Aujourd'hui, l'industrie pharmaceutique reconnaît des avantages notables associés à l'usage unique:
- la sécurité, du fait de la limitation du risque de contamination croisée,
- et les gains de productivité obtenus (i) par réduction des coûts de fonctionnement, notamment des frais de personnel ; (ii) par mise en oeuvre d'une culture à haute densité contribuant à limiter les temps de culture et l'occupation au sol; et (iii) par une mise en échelle industrielle plus rapide et moins coûteuse.

Traditionnellement les bio réacteurs mis en oeuvre sont réutilisables, en verre ou en cuve inox, nécessitant leur nettoyage, stérilisation et revalidation constante. Ces opérations, de plus en plus coûteuses suite à la croissance continue des exigences réglementaires et de qualité, sont éliminées par l'implantation de solutions à usage unique.

Il existe une demande croissante pour des équipements tels que des bioréacteurs fermenteurs, cuve de stockage et préparation «upstream & downstream» («flux montants & flux descendants») à usage unique. Or de tels systèmes sont nécessairement équipés de capteurs prévus pour mesurer des paramètres physico-chimiques, biochimiques ou biophysiques. On peut notamment citer des capteurs réalisant une spectroscopie d'impédance, en particulier les capteurs de technologie capacitive conçus par le présent déposant.

Ces capteurs capacitifs permettent de détecter des variations relatives de capacités de 10⁻⁵ dans des milieux de faible résistance (quelques dizaines d'Ohms). Cette technique permet également, grâce à la spectroscopie d'impédance, de déterminer les paramètres des courbes de β dispersion (radio fréquence). Les mesures fournissent alors non seulement une concentration de cellules vivantes mais aussi des indications sur la morphologie et l'état physiologique des cellules. Les sondes de mesures usuelles sont de taille macroscopique et destinées à des cuves de fermentation en inox. Elles exploitent typiquement des configurations à 4 électrodes pour s'affranchir des effets de double couche de polarisation.

On peut notamment citer des capteurs capacitifs spécifiquement dédiés aux mesures de conductance, en particulier pour des applications en purification de type «downstream». Ces capteurs de conductance peuvent présenter les mêmes géométries et structures d'électrode que des capteurs capacitifs permettant des mesures complètes d'impédance avec mesures de capacitance et de conductance. On connaît aussi l'utilisation d'un connecteur aseptique qui permet de rentrer des capteurs solides dans un fermenteur jetable à condition d'être dans un environnement très protégé et sous hotte à flux laminaire. Cette solution est globalement plus coûteuse en raison du prix de revient et de fonctionnement des capteurs solides: décontamination, démontage, nettoyage, montage du capteur. Par ailleurs, les capteurs solides prévus pour équiper des bioréacteurs à usage unique doivent être stérilisés avant chaque utilisation, ce qui ne limite pas en soi le risque de contamination liée à l'enchainement de tâches nécessaires.

L'article Davey, C. L. et al. : "Real-time monitoring of the accretion of Rhizopus oligosporus biomann during the solid-substrate tempe fermentation" in World Journal of Microbiology and Biotechnology, Rapid Communications of Oxford, Oxford (GB), 1991, p. 248-249, présente un incubateur dans lequel sont placées des boîtes de Pétri. Des capteurs, tels que des capteurs de température, un capteur de PH et un capteur de Bugmeter avec quatre électrodes pour mesurer la capacité, sont placés dans les boîtes de Pétri. Les parois de l'incubateur sont traversées par les lignes de signaux des capteurs, qui transmettent les signaux détectés par les capteurs à des appareils de traitement de données situés à l'extérieur de l'incubateur.

Il est connu du document WO 2005/068059 A1 une vessie de mélange destinée à être utilisée dans des procédés techniques biologiques. Dans la vessie de mélange, qui présente une enveloppe extérieure souple, un fluide est reçu et agité en utilisant un élément d'agitation disposé dans la vessie de fluide. L'élément d'agitation est entraîné par un dispositif d'entraînement situé à l'extérieur de la vessie de mélange. La vessie peut comporter un distributeur de gaz intégral et un logement de capteur.

On connaît du document FR 2 867 278 A1 un procédé et un dispositif de mesure en ligne et de caractérisation en ligne d'une biomasse dans un processus de fermentation avec des bactéries lactiques. Le FR 2 867 278 A1 divulgue à cet égard l'agencement d'un capteur capacitif dans un récipient recevant directement un liquide.

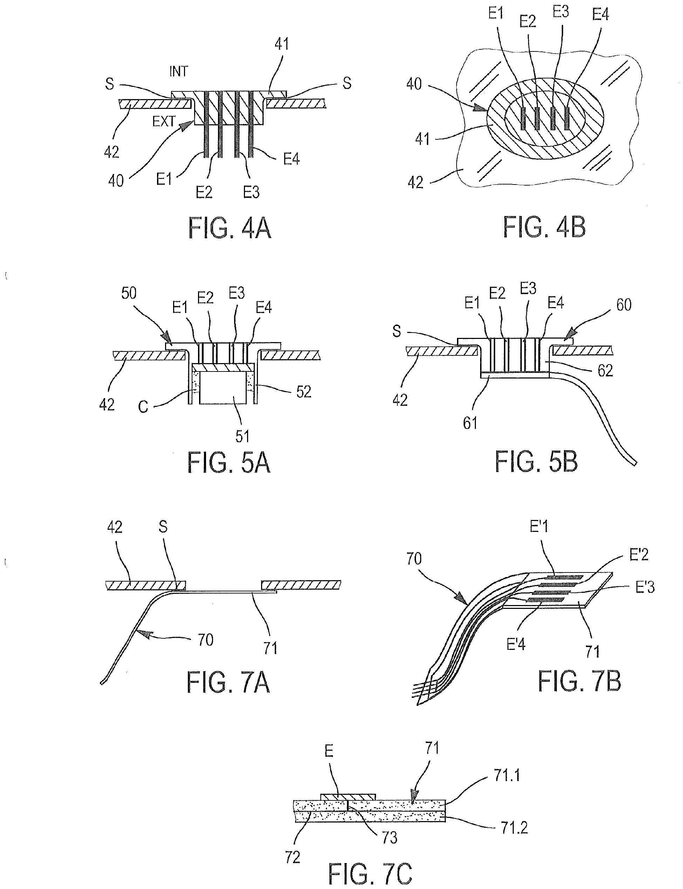

L'article Carvell, John P., Dowd, Jason E. : "On-line measurements and control of viable cell density in cell culture manufacturing processes using radio-frequency impédance" in Springer-Verlag : "Cytotechnology" (2006) 50 : p. 35-48, présente, à la figure 5, un bioréacteur dont la paroi du boîtier est traversée par des capteurs individuels qui plongent dans le liquide contenu dans le bioréacteur. Dans cette divulgation, le bioréacteur lui-même sert de récipient pour recevoir le liquide à traiter.

On connaît du document WO 02/102961 A2 une combinaison de processus de fermentation en continu et par lots. Les processus de fermentation qui y sont décrits servent à produire de l'alcool potable. L'imprimé divulgue une plaque de tête de bioréacteur sur laquelle sont soudées des têtes de lecture et de sélection. En outre, une vanne à membrane destinée à prélever des échantillons dans le bioréacteur est soudée à la paroi du bioréacteur.

Le but de la présente invention est de remédier à ces inconvénients en proposant un bioréacteur ou conteneur à usage unique intégrant un capteur capacitif qui présente des performances suffisantes pour des applications en bioréacteur, tout en ayant un coût de réalisation et un coût de fonctionnement minimisés.

La présente invention atteint cet objectif avec un bioréacteur ou conteneur ayant toutes les caractéristiques de la revendication 1. Une forme de réalisation d'und bioréacteur ou contenteur à usage unique selon la présente invention comprend une enceinte prévue pour contenir un milieu biologique, et au moins un dispositif pour capter au moins une mesure d'impédance dans un processus physique, physico-chimique et/ou biologique dans ledit milieu, caractérisé en ce que ce dispositif capteur est fixé au travers d'une paroi de ladite enceinte de façon à en être partie intégrante et comprend une partie de captation en contact direct avec l'intérieur dudit bioréacteur ou conteneur et une partie de connexion s'étendant vers l'extérieur de ladite enceinte.

Pour réaliser l'invention, il est proposé un dispositif à usage unique pour capter au moins une mesure d'impédance dans un processus physique, physico-chimique et/ou biologique, comprenant des moyens pour réaliser au moins une mesure d'impédance dans un processus physique, physico-chimique et/ou biologique dans ledit milieu. Ce dispositif est agencé pour être fixé au travers d'une paroi d'une enceinte prévue pour contenir un milieu biologique, de façon à en être partie intégrante, et en ce qu'il comprend une partie de captation en contact direct avec ledit milieu biologique et une partie de connexion s'étendant vers l'extérieur de ladite enceinte.

Selon la présente invention ce dispositif capacitif à usage unique est spécifiquement dédié à la mesure de biomasse vivante dans un milieu biologique, au moyen de la mesure de capacitance.

Le dispositif capteur à usage unique en lien avec l'invention peut être associé en ligne à d'autres capteurs physiques, physico-chimiques ou biochimiques à usage unique tel que des capteurs de température, pH, concentration en oxygène dissous, concentration en CO₂ dissous, densité optique, pression, niveau, concentration en ammonium, en glycérol, taux de lactate et glucose, et plus généralement tout autre capteur à usage unique dans le but d'améliorer la connaissance et le contrôle d'une culture biologique.

Un capteur optique utilisant une pastille fluorescente à usage unique est une technique avantageuse de couplage et concerne la mesure en ligne de paramètres tels que le pH, la concentration en oxygène dissous et la mesure de la concentration en CO₂ dissous. Il peut ainsi intégrer plusieurs capteurs à usage unique sur un même support.

Ainsi avec l'invention, il devient possible de réaliser le premier système multicapteur à usage unique en ligne, biocompatible et stérilisable, mesurant les étapes clés d'une culture cellulaire, en vue de la piloter selon des critères et des objectifs quantitatifs et qualificatifs de production à toute échelle. Il permettra également de coupler le bio-diagnostic et les microtechnologies pour la caractérisation de milieux biologiques divers.

Dans un mode particulier de réalisation, le dispositif capteur est agencé pour être installé dans une enveloppe stérile à applications multiples.

Le dispositif capteur peut aussi être agencé pour être inséré et plongeant dans une enceinte prévue pour contenir des cellules biologiques.

Selon l'invention, le dispositif capteur à usage unique est agencé pour être installé dans un bioréacteur réalisé pour une utilisation en lit fixe, c'est-à-dire tout type de culture de cellules sur support adhérent, micro-porteur et macro-porteur ou toute autre application dans laquelle les mesures recherchées sont issues d'un procédé de culture de typé adhérent. Il peut par exemple être inséré dans un panier plongeant dans une enceinte prévue pour contenir des cellules biologiques dont le modèle de croissance est considéré statique, par exemple, pour la culture de cellules souches dans le but d'applications médicales.

Dans une autre variante de l'invention, le dispositif capteur à usage unique selon l'invention est agencé pour être installé dans un bioréacteur à usage unique.

Le dispositif capteur selon l'invention peut aussi être inséré dans un passage aménagé dans une paroi du bioréacteur et présenter une face de captation orientée et en contact avec l'intérieur de ce bioréacteur.

Dans une forme de réalisation avantageuse, le dispositif capteur à usage unique comprend des électrodes s'étendant sur la partie de captation.

Il peut être réalisé sous la forme d'une pièce mécanique usinée agencée pour recevoir sur une partie de captation les électrodes et sur une partie de connexion des moyens de sortie de signal de mesure.

Il peut aussi être réalisé sous la forme d'une pièce moulée par injection incluant sur une partie de captation les électrodes et sur une partie de connexion les moyens de sortie de signal de mesure.

Les moyens de sortie de signal de mesure peuvent comprendre un connecteur ou bien un câble ou un flexible.

Les électrodes peuvent être réalisées à partir de pièces métalliques ou par dépôt métallique sur un support isolant qui peut être un flexible.

Ce support flexible peut être réalisé sous la forme de deux films isolants dont au moins un supporte une électrode réalisée par dépôt et reliée électriquement à un conducteur disposé entre les deux films isolants.

Le dispositif capteur à usage unique peut être agencé pour être installé dans une enveloppe stérile à applications multiples.

Dans une variante particulière de réalisation, le dispositif capteur à usage unique comprend des moyens embarqués pour traiter localement des signaux de mesure d'impédance. Ces moyens embarqués peuvent être prévus pour produire un signal d'alerte en réponse à une détection d'anomalie par les moyens de traitement embarqués.

Suivant un autre aspect de l'invention, il est proposé un procédé pour réaliser un dispositif capteur à usage unique selon l'invention, mis en oeuvre dans un environnement de production répondant aux critères d'asepsie et de stérilité requis pour l'utilisation d'un bioréacteur.

En effet, toutes les techniques mises en oeuvre (soudure, lavage, nettoyage, décontamination bactérienne et virale, outils mécaniques, moyens d'analyse) représentent une méthodologie nouvelle de production qui jusque là n'avait pas était mise en oeuvre pour la production de capteurs solides et traditionnels.

Les matériaux mis en oeuvre pour la réalisation du dispositif capteur à usage unique sont nécessairement biocompatibles. Ces matériaux peuvent avantageusement être compatibles pour une stérilisation par gamma-irradiation avant et/ou après l'installation du dispositif capteur sur le bioréacteur.

La présente invention est aussi applicable à des procédés de mesure hors ligne tels que du diagnostic à usage médical, du contrôle qualité biologique, médical, environnemental, ou de l'emballage instrumenté spécifique pour le contrôle biologique, et n'est donc pas limitée aux seules enceintes stériles avec mesure en ligne.

Dans une autre application d'un capteur de biomasse à usage unique, celui-ci est monté sur une enceinte aseptique et est éventuellement associé à un capteur physique ou biologique, pour réaliser un conteneur de stockage de milieu biologique jetable sur lequel sont intégrés des capteurs jetables. Ces derniers permettent à tout moment de contrôler la biomasse vivante et d'autres paramètres physico-chimiques avec d'autres capteurs associés.

Ce moyen de stockage peut être réalisé à partir des mêmes capteurs et matériaux d'enceinte que pour l'application bioréacteur. Il permet de détecter tout incident biologique pendant le stockage de la matière, sans risque de perte de stérilité pendant ce contrôle car il n'y pas de prise d'échantillon.

Cette application est particulièrement indiquée pour le contrôle industriel de matière biologique ou chimique avant et après livraison dans le cadre d'un suivi de qualité du produit. Elle procure également un avantage certain pour toute conservation de longue durée pour laquelle il est nécessaire d'avoir un contrôle régulier de la qualité ou d'estimer la péremption d'un produit tel que les denrées alimentaires.

Il devient ainsi possible de réaliser un emballage intelligent permettant de détecter, au moyen de ses capteurs intégrés, toute anomalie ou accident de conservation, et de procurer une traçabilité du transport de compositions, de produits ou de denrées impliquant des exigences de sécurité et de qualité.

D'autres avantages et caractéristiques de l'invention apparaîtront à l'examen de la description détaillée d'un mode de mise en oeuvre nullement limitatif, et des dessins annexés sur lesquels :
- La figure 1 est une vue schématique d'un capteur à usage unique mis en oeuvre dans un réacteur à usage unique en lit fixe;
- La figure 2 est une vue schématique d'un bioréacteur à usage unique, qui n'appartient pas à la présente inevention, pour cultures de cellules en suspension équipé de capteurs à usage unique;
- La figure 3 illustre un exemple pratique d'un bioréacteur à usage unique équipé de capteurs à usage unique;
- La figure 4A est une vue en coupe d'un capteur à usage unique à quatre électrodes;
- La figure 4B est une vue de dessus du capteur à usage unique de la figure 4A;
- La figure 5A est une vue en coupe d'un premier mode de connexion des électrodes d'un capteur à usage unique réalisé à partir d'une pièce usinée et mettant en oeuvre un connecteur enrobé d'une colle;
- La figure 5B est une vue en coupe d'un second mode de connexion des électrodes d'un capteur à usage unique réalisé à partir d'une pièce usinée et mettant en oeuvre un câble ou un flexible;
- La figure 6A est une vue en coupe d'un capteur à usage unique pourvu d'électrodes et d'un connecteur surmoulés par injection;
- La figure 6B est une vue en coupe d'un capteur à usage unique pourvu d'électrodes et d'un câble ou flexible surmoulés par injection;
- Les figures 7A, 7B et 7C illustrent un exemple particulier d'un capteur à usage unique dans lequel les électrodes sont déposées directement sur un flexible;
- La figure 8 illustre une combinaison d'un capteur à usage unique de mesure d'impédance et d'un capteur à usage unique de mesure de fluorescence; dans un montage séparé, et
- La figure 9 illustre la combinaison d'un capteur à usage unique de mesure d'impédance et d'un capteur à usage unique de mesure de fluorescence, montés sur un même support.

On va maintenant décrire, en référence aux figures précitées, plusieurs exemples de capteurs à usage unique, dans différentes configurations de mise en oeuvre.

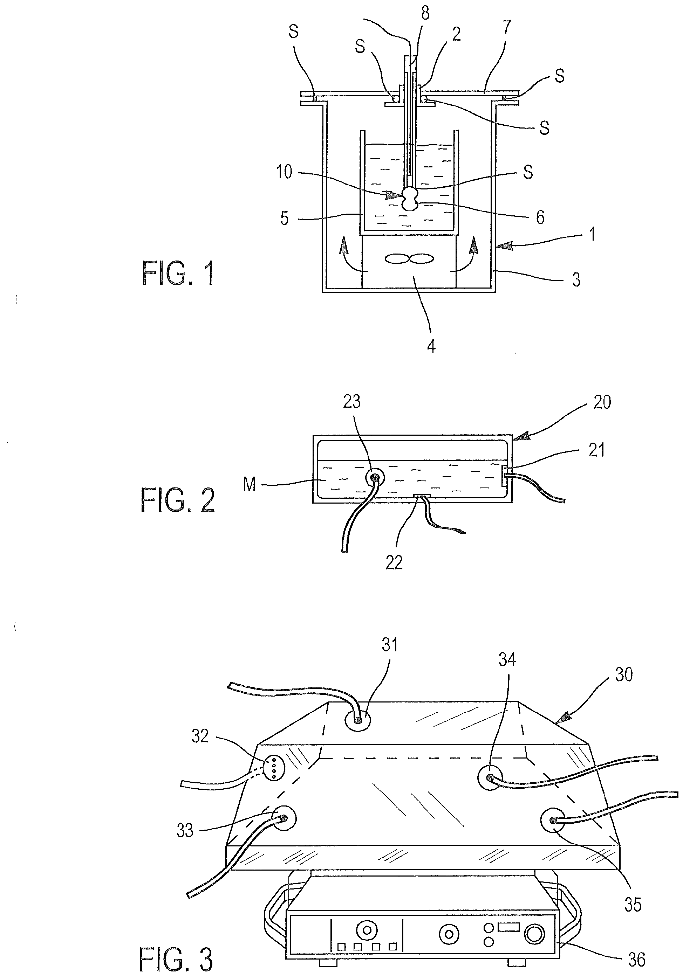

On va en premier lieu décrire un exemple de mise en oeuvre d'un capteur d'impédance à usage unique 10 mis en oeuvre dans un bioréacteur réutilisable 1, en référence à la figure 1. Ce bioréacteur 1 peut, à titre d'exemple non limitatif, être du type de ceux commercialisés par les sociétés WAVE, SARTORIUS ou ATMI.

Le capteur d'impédance mis en oeuvre peut être du type capacitif tel que celui commercialisé par le présent déposant et divulgué dans le document FR2 812 725. Il peut aussi être du type «multicapteur», comme ceux incluant des capteurs optiques qui sont divulgués dans le document FR2 867 279 au nom du présent déposant.

Le bioréacteur 1 comprend, dans une enceinte 3, un lit fixe 5 contenant des cellules biologiques et équipé d'un agitateur 4, un couvercle 7 fixé à l'enceinte 3 par des soudures S et percé d'un orifice 2 dans lequel est inséré un tube récepteur 9 prévu pour recevoir un capteur d'impédance à usage unique 10 pourvu à son extrémité d'un jeu d'électrodes 6. Ces électrodes peuvent être au nombre de deux, quatre ou plus, en fonction de la technique de mesure mise en oeuvre. Le capteur d'impédance 10 comporte des conducteurs 8 pour délivrer les signaux de mesure générés par ce capteur.

Lorsqu'il s'agit d'équiper un bioréacteur à usage unique 20, en référence à la figure 2, des capteurs à usage unique 21, 22, 23 peuvent être installés respectivement sur une face latérale, le fond et la face avant de la cuve de ce bioréacteur. Ainsi, comme l'illustre la figure 3, un bioréacteur à usage unique 30 mis en oeuvre sur un appareil de contrôle 36 à usage permanent peut être fourni entièrement équipé de capteurs à usage unique 31-35.

En référence aux figures 4A et 4B, un capteur de mesure d'impédance unique 40 peut être disposé dans un orifice d'une paroi 42 d'un bioréacteur, avec une partie de captation dont une face de captation 41 est disposée à l'intérieur du bioréacteur et fixée par soudure S à la face intérieure de cette paroi. Le capteur de mesure d'impédance fait alors partie intégrante du bioréacteur.

On peut aussi prévoir d'autres modes de fixation tels que le vissage, l'emmanchage ou le collage. Le capteur 40 est pourvu sur la face de captation 41 de quatre électrodes E1-E4, dont deux assurent une fonction d'excitation et deux une fonction de mesure.

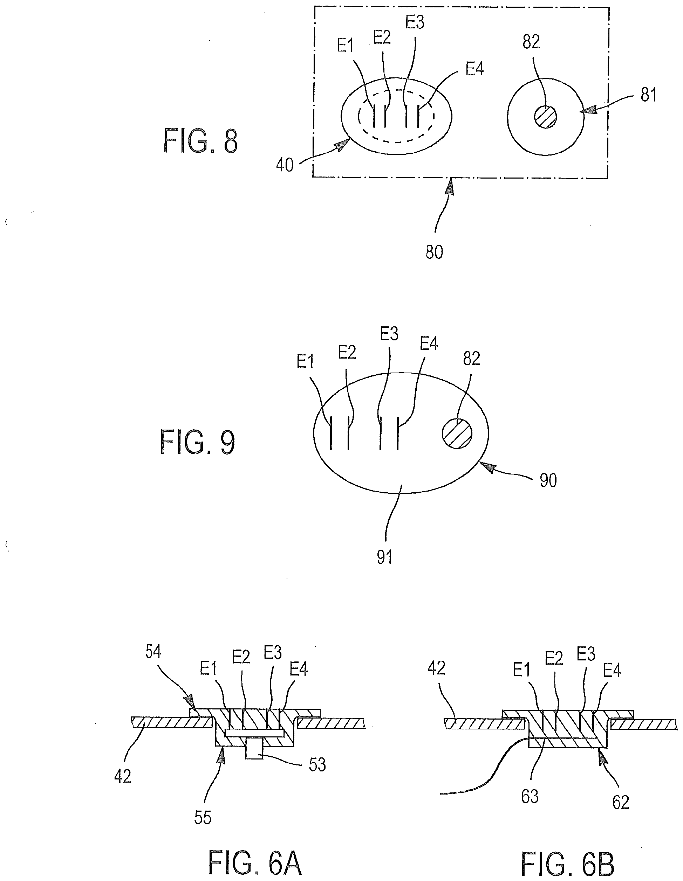

La connexion des électrodes d'un capteur à usage unique à un appareil de mesure ou de contrôle peut être effectuée, soit au moyen d'un connecteur (figures 5A et 6A), soit au moyen d'un câble ou flexible (figures 5B et 6B). Ces moyens de connexion constituent la partie de connexion du capteur à usage unique.

Le capteur à usage unique peut être réalisé soit à partir d'une pièce en matière plastique usinée (figures 5A et 5B), soit par injection avec surmoulage des électrodes et des moyens de connexion (figures 6A et 6B).

Les électrodes d'un capteur de mesure d'impédance à usage unique peuvent être réalisées :
- soit sous la forme de pièces métalliques qui peuvent être collées (figures 4A, 4B, 5A, et 5B) ou surmoulées (figures 6A et 6B),
- soit par déposition, par exemple par des techniques de dépôt sous vide, par sérigraphie, lithographie, ou électrolyse.

Dans une première configuration illustrée par la figure 5A, le capteur à usage unique 50 inséré dans un orifice d'un bioréacteur est réalisé à partir d'une pièce usinée 52 dans laquelle ont été disposés quatre électrodes E1 - E4 et un connecteur51 fixé à la pièce usinée par collage C.

Dans une seconde configuration illustrée par la figure 5B, le capteur à usage unique 60, réalisé à partir d'une pièce usinée 62, est équipé d'un flexible ou d'un câble 61 relié électriquement aux quatre électrodes E1 - E4.

Dans une troisième configuration illustrée par la figure 6A, le capteur à usage unique 55 est réalisé par injection de matière plastique avec surmoulage des quatre électrodes E1 - E4 et d'un connecteur 53.

Dans une quatrième configuration, le capteur à usage unique 62 est réalisé par injection de matière plastique avec surmoulage des quatre électrodes E1 - E4 et d'un flexible 63.

Dans un autre mode de réalisation illustré par les figures 7A, 7B et 7C, les électrodes E'1 - E'4 d'un capteur à usage unique 70 sont réalisées par dépôt métallique directement sur un flexible 71. Ce capteur 70 peut ensuite être fixé par soudure S sur la paroi 42 d'un bioréacteur.

En référence à la figure 7C, le flexible 71 est réalisé par superposition de deux flexibles isolants 71.1, 71.2 entre lesquels est réalisée une piste conductrice 72 reliée à une électrode déposée E au moyen d'un via 73 traversant le flexible isolant 71.1 sur la face extérieure duquel est déposée l'électrode E.

Un capteur de mesure d'impédance à usage unique 40 peut être combiné à un autre capteur à usage unique, par un exemple un capteur de fluorescence pour la mesure du pH, de l'oxygène (O₂) ou du gaz carbonique (CO₂).

Les deux capteurs à usage unique peuvent être soit montés séparément (figure 8), soit disposés sur un même support (figure 9). Dans le premier mode, chaque capteur à usage unique 40, 82 est fourni sur un support distinct 41, 81, tandis que dans le second mode, les éléments actifs des deux capteurs - les quatre électrodes E1 - E4 du capteur de mesure d'impédance, et le capteur de fluorescence 82 - sont disposés sur un même patch ou support 91 et constituent un double capteur à usage unique 90.

On va maintenant décrire des contraintes de réalisation de capteurs à usage unique selon l'invention. Selon les règles de bonnes pratiques de production (BPF ou cGMP), les solvants et toutes pollutions sont à proscrire des procédés de fabrication pour éviter de polluer les bio-matériaux utilisés dans la réalisation industrielle du capteur. En particulier, la norme "leachables and extractables" («relargables et extractibles») (21 CFR part 600.11) doit être suivie.

La chaîne de stérilisation ne doit pas être rompue entre la fabrication d'un capteur et son implantation chez un sous-traitant. Les capteurs à usage unique sont envoyés dans un conditionnement stérile et sont montés sous un flux laminaire sur la poche.

Une fois que tous les éléments sont montés, la poche est stérilisée par rayonnement gamma.

Les matériaux utilisés doivent bien sûr être bio-compatibles et résistants à la dose de rayon gamma mise en oeuvre.

Le capteur à usage unique doit être pré-décontaminé, i. e. être débarrassé de tout polluant sous un seuil accepté par l'application. Ces polluants sont principalement capturés lors des processus utilisés pour la fabrication du capteur (usinage, montage, soudage, collage...). L'emballage des capteurs à usage unique est étudié pour conserver la stérilité du capteur et pour être compatible aux exigences et contraintes pour une utilisation en salle propre. Ainsi, un emballage à double parois est préconisé.

Le capteur est monté sur le bioréacteur et la stérilisation gamma est effectuée préférentiellement après le montage du capteur et de tous les constituants (tuyaux, bouchons, raccords,...). Pour d'autres applications, la stérilisation pourra être réalisée juste après l'emballage du capteur. D'autres moyens de stérilisation peuvent être également envisagés.

Les capteurs à usage unique peuvent être connectés, par voie filaire ou non filaire, à des équipements électroniques de mesure d'impédance disponibles sur le marché ou à des équipements plus spécifiques à la mesure de biomasse tel que celui divulgué dans le document FR2 812 725.

On peut aussi prévoir que tout ou partie de l'électronique de mesure et de traitement soit embarquée sur le bioréacteur, l'emballage ou le conteneur intelligent, en utilisant des technologies d'intégration, éventuellement avec autoalimentation électrique. En particulier, des dispositifs d'alerte, optiques ou sonores, peuvent être aussi associés aux capteurs à usage unique selon l'invention. Par exemple dans le cas d'un emballage intelligent prévu pour stocker un milieu biologique, un signal d'alerte peut être généré lorsqu'une évolution anormale de la capacitance ou d'une grandeur physique ou physico-chimique est détectée. On peut notamment se référer au document FR2 874 26 au nom du présent déposant, qui divulgue un procédé et dispositif de détermination de biomasse dans un milieu.

Des techniques de fabrication issues de l'électronique et de la microélectronique pourraient être mises en oeuvre pour une fabrication à grande échelle d'électrodes d'un capteur à usage unique selon l'invention. En particulier, un circuit intégré de détection capacitive pourrait être réalisé par des techniques de diffusion, par exemple sur des substrats polymères ou des substrats couramment employés pour la réalisation de microsystèmes

## Revendications

1. Bioréacteur ou conteneur (1) comprenant une enceinte (3) prévue pour contenir un milieu biologique et comprenant au moins un dispositif à usage unique pour capter au moins une mesure d'impédance dans un processus physique, physico-chimique et/ou biologique dans ledit milieu, ce dispositif capteur étant spécifiquement dédié à la mesure de biomasse vivante dans un milieu biologique, au moyen de la mesure de capacitance,
ce dispositif capteur étant fixé au travers d'une paroi (7) de ladite enceinte (3) de façon à en être partie intégrante et comprenant une partie de captation en contact direct avec l'intérieur dudit bioréacteur ou conteneur (1) et une partie de connexion s'étendant vers l'extérieur de ladite enceinte, cette paroi (7) étant percé d'un orifice (2) dans lequel est inséré un tube récepteur (9) prévu pour recevoir le dispositif capteur d'impédance à usage unique (10) pourvu à son extrémité d'un jeu d'électrodes (6), la paroi (7) sur laquelle est fixé le dispositif capteur (10) étant un couvercle (7),
**caractérisé en ce que** le bioréacteur ou conteneur (1) comprend en plus dans l'enceinte (3) un lit fixe (5) espacé de l'enceinte, et **en ce que** le couvercle (7) est fixé à l'enceinte restante par des soudures (S).

2. Bioréacteur ou conteneur selon la revendicaton 1,
**caractérisé en ce que** le tube récepteur (9) est fixé à la paroi (7), particulièrement au convercle (7), par des soudures (S).

3. Bioréacteur ou conteneur selon l'une quelconque des revendications précédentes,
**caractérisé en ce que** le dispositif capteur (10) comporte des conducteurs (8) pour délivrer les signaux de mesure générés par le dispositif capteur (10).

4. Bioréacteur ou conteneur selon l'une quelconque des revendications précédentes,
**caractérisé en ce que** le lit fixe (5) contient des cellules biologiques, en particulier des cellules vivantes.

5. Bioréacteur ou conteneur selon l'une quelconque des revendications précédentes,
**caractérisé en ce qu'**il s'agit d'un bioréacteur ou conteneur réutilisable.

6. Bioréacteur ou conteneur selon l'une des revendications 1 à 4, **caractérisé en ce qu'**il s'agit d'un bioréacteur ou conteneur à usage unique.

7. Bioréacteur ou conteneur selon l'une quelconque des revendications précédentes,
**caractérisé en ce que** tout ou partie de l'électronique de mesure et de traitement est embarquée sur le bioréacteur.

## Patentansprüche

1. Bioreaktor oder Behälter (1) mit einer Umhüllung (3), die zur Aufnahme eines biologischen Mediums vorgesehen ist und mindestens eine Einwegvorrichtung zur Erfassung mindestens einer Impedanzmessung bei einem physikalischen, physikalisch-chemischen und/oder biologischen Prozess in diesem Medium umfasst, wobei diese Sensorvorrichtung speziell zur Messung lebender Biomasse in einem biologischen Medium bestimmt ist, mittels Kapazitätsmessung, wobei diese Sensorvorrichtung durch eine Wand (7) der Umhüllung (3) befestigt ist, so dass sie ein integraler Teil davon ist, und einen Aufnahmeteil in direktem Kontakt mit dem Inneren des Bioreaktors oder Behälters (1) und einen Verbindungsteil, der sich nach außen von der Umhüllung erstreckt, umfasst, wobei diese Wand (7) von einer Öffnung (2) durchbrochen ist, in die ein Aufnahmerohr (9) eingeführt ist, das zur Aufnahme der Einweg-Impedanz-Sensorvorrichtung (10) vorgesehen ist, die an ihrem Ende mit einem Satz Elektroden (6) versehen ist, wobei die Wand (7), an der die Sensorvorrichtung (10) befestigt ist, eine Abdeckung (7) ist,
**dadurch gekennzeichnet, dass** der Bioreaktor oder Behälter (1) zusätzlich in der Umhüllung (3) ein festes Bett (5) umfasst, das mit Abstand von der Umhüllung angeordnet ist, und dass die Abdeckung (7) durch Schweißnähte (S) an der verbleibenden Umhüllung befestigt ist.

2. Bioreaktor oder Behälter nach Anspruch 1,
**dadurch gekennzeichnet, dass** das Aufnahmerohr (9) an der Wand (7), insbesondere an der Abdeckung (7), durch Schweißnähte (S) befestigt ist.

3. Bioreaktor oder Behälter nach einem der vorhergehenden Ansprüche,
**dadurch gekennzeichnet, dass** die Sensorvorrichtung (10) Leiter (8) zur Abgabe der von der Sensorvorrichtung (10) erzeugten Messsignale aufweist.

4. Bioreaktor oder Behälter nach einem der vorhergehenden Ansprüche,
**dadurch gekennzeichnet, dass** das feste Bett (5) biologische Zellen, insbesondere lebende Zellen, enthält.

5. Bioreaktor oder Behälter nach einem der vorhergehenden Ansprüche, **dadurch gekennzeichnet, dass** es sich um einen wiederverwendbaren Bioreaktor oder Behälter handelt.

6. Bioreaktor oder Behälter nach einem der Ansprüche 1 bis 4,
**dadurch gekennzeichnet, dass** es sich um einen Einweg-Bioreaktor oder -Behälter handelt.

7. Bioreaktor oder Behälter nach einem der vorhergehenden Ansprüche, **dadurch gekennzeichnet, dass** die gesamte oder ein Teil der Mess- und Verarbeitungselektronik an Bord des Bioreaktors ist.

## Claims

1. Bioreactor or container (1) comprising an enclosure (3) intended to contain a biological medium and comprising at least one disposable device for capturing at least one impedance measurement in a physical, physico-chemical and/or biological process in said medium, this sensor device being specifically dedicated to the measurement of living biomass in a biological medium, by means of capacitance measurement, this sensor device being fixed through a wall (7) of said enclosure (3) so as to be an integral part thereof and comprising a collection portion in direct contact with the inside of said bioreactor or container (1) and a connection portion extending to the outside of said enclosure, this wall (7) being pierced by an orifice (2) into which is inserted a receiving tube (9) designed to receive the disposable impedance sensor device (10) provided at its end with a set of electrodes (6), the wall (7) to which the sensor device (10) is attached being a cover (7), **characterized in that** the bioreactor or container (1) additionally comprises in the enclosure (3) a fixed bed (5) spaced from the enclosure, and **in that** the cover (7) is attached to the remaining enclosure by welds (S).

2. Bioreactor or container according to claim 1,
**characterized in that** the receiving tube (9) is secured to the wall (7), particularly to the cover (7), by welds (S).

3. Bioreactor or container according to any of the preceding claims, **characterized in that** the sensor device (10) comprises conductors (8) for outputting the measurement signals generated by the sensor device (10).

4. Bioreactor or container according to any of the preceding claims, **characterized in that** the fixed bed (5) contains biological cells, in particular living cells.

5. Bioreactor or container according to any of the preceding claims, **characterized in that** it is a reusable bioreactor or container.

6. Bioreactor or container according to any one of claims 1 to 4, **characterized in that** it is a single-use bioreactor or container.

7. Bioreactor or container according to any of the preceding claims, **characterized in that** all or part of the measurement and processing electronics is on-board the bioreactor.
